# EUROPEAN PATENT SPECIFICATION

(11) **EP 4 082 426 B1**
(45) Date of publication and mention of the grant of the patent: **26.07.2023**
(21) Application number: 21171562.8
(22) Date of filing: 30.04.2021
(51) Int. Cl.: A61B 5/00, H02J 50/00

(54) **SELF-SUFFICIENT SIGNAL MONITOR**
AUTARKER SIGNALMONITOR
MONITEUR DE SIGNAL AUTO-SUFFISANT

(43) Date of publication of application: 02.11.2022
(73) Proprietor: Celtro GmbH, 01099 Dresden (DE)
(72) Inventor: BUDNY, Jarek, 01067 Dresden (DE); PIORKOWSKI, Judith, 01099 Dresden (DE); TEEPE, Gerd, 01099 Dresden (DE)
(74) Representative: Maikowski & Ninnemann Patentanwälte Partnerschaft mbB

(56) References cited:
- WO-A1-2015/117089
- WO-A1-2021/089531

## Description

The invention discloses a device, which comprises a multiple of microneedles and a chip comprising at least one comparator with adaptive level, sequence control circuit, at least one capacitor stack built by n capacitors and 2n switches, at least one buffer capacitor outside the at least one capacitor stack, at least two additional switches outside the at least one capacitor stack, a CMOS-Logic, wherein further, the device comprises an interposer layer comprising holes for the multiple of microneedles, at least one sensor and a lid. The chip of the device is located on one surface of the interposer layer and that the lid and the interposer layer form a capsule for the chip and are connected to at least one transmitter coil. On and inside the capsule the device contains at least one sensor to measure signals such as electrical fields, motion, pressure, temperature, and light. Further, each microneedle of the array of microneedles has a distal end which protrudes from the chip and the device is adapted to be electrically self-sufficient.

At a high level, monitoring follows this path:
Sensing. Sensing means collection of environmental or biological parameters such as temperature, pressure, geometric coordinates (location), concentration of chemical substances, electrical, magnetic or electromagnetic fields in strength and orientation, and such like. Typically sensing requires a transformation of the measured component into a signal in proportion of the original component. Today most of those "signals" are electrical in nature and can be embodied in a microchip, which is today one of the cheapest ways to capture (sense) and process signals.

Storing. Once a signal is acquired, it needs to remain present in some form for further processing. In a microchip, a signal can be acquired through analog-to-digital conversion and subsequent storage in digital memories such as SRAM.

Evaluating. Evaluation means interpreting this signal or in combination with other signals with the purpose to derive a conclusion. As an example, the level of a water tank is monitored and an acquired signal is a representation of the water level. If the signal is compared to a reference, then a warning can be issued once the water level is higher than the reference. In this sense, a data compression takes place, as the system does not need to provide subsequent water levels, but only issue a warning message once a critical level is exceeded. In this sense evaluation is the mechanism to interpret data within a predefined context. It goes in hand with data reduction. In the above example of a water tank, out of a multitude of signals one single bit is being generated to indicate a warning level is reached.

Transmission. The evaluated data needs to reach a destination. Any signal transmission technology is normally suited to transport the signal from its monitoring location to where it is needed. Today, mostly electromagnetic signaling is used over wires or wireless. Signal transmission however also needs energy. Claude Shannon formulated in 1948 the basic theory on minimum energy requirements for data transmission. Autonomous systems must operate within these limits.

Monitoring devices can be used in a wide range of applications. For example monitoring of environmental parameters such as temperature, pressure, geometric coordinates (location), concentration of chemical substances e.g. oxygen, CO₂, Nitrogen, or hazardous substances like CO, CH₄, NH₃ and such like, electrical, magnetic or electromagnetic fields in strength and orientation, motion and such like are of interest.

In addition, monitoring devices are becoming more and more common in biological signal recording. For example, continuous biological signal recording and timely therapy implementation are becoming increasingly relevant to guide and manage clinical patient care. Typical biological signals utilized in today's medicine encompass (i) electrical field information (e.g., ECGs and EEGs), (ii) information on singular organ motion (e.g., heart and inner ear) and whole-body motion, (iii) information on pressure (e.g., blood pressure, pressure in gastrointestinal organs, and intracerebral pressure), (iv) temperature information, and (v) light source derived information (e.g., level of oxygen saturation in the blood, level of blood sugar).

Timely knowledge of these data enables physicians to quickly react on patients' deterioration. This has been shown to increase life quality and prolong life expectancy. Continuous collection, storage and transmission of such data over long time periods are prerequisites for advanced remote patient management and future digital health applications.

Biological signal recording has evolved with the introduction of implantable medical electronic devices. Electrical field information (ECG signals) obtained out of cardiac pacemakers where among the first biological signals which were routinely and continuously recorded, stored and utilized for clinical decision making. Later remote data transmission capabilities and infrastructure have enabled automatized signal collection, data transmission and timely and remote medical review. This has paved the way for the development of a multitude of wearable and implantable purely diagnostic sensor devices for medical purposes.

A further application field is the monitoring of biological signal in wildlife. Numerous opportunities exist in this field, for example from monitoring the biological functions of wild animals to monitoring their movement patterns in the wild. Further, monitoring of biological signals is also applied in plants. E.g. monitoring the condition of plants in agriculture is an interesting field of application

However, monitoring requires energy and provision of energy is a major problem. Either there has to be a connection to a power grid, such as mains or a battery has to be provided. Both methods have drawbacks. Connection to the power grid requires infrastructure in the form of wires. Also it requires the power grid to which it is connected to be reliably present. In some cases, connections are impossible to achieve if it comes to mobile devices or the body of a living being (which is also mobile) which should be monitored.

One possibility is energy storage, which is enabled by accumulators or batteries. However, this is always a temporary solution, as batteries need replacement and accumulators recharge. In this sense regular maintenance is required if the service is to be maintained reliably. Replacement and/or recharging of accumulators or batteries may cause major problems, for example if the sensor for monitoring is attached to a living being.

In this context, today's diagnostic sensor devices (such as the one disclosed in WO 2015/117089 A1) are limited by their dependency on external energy sources. As an example, so called implantable loop recorders (e.g., Medtronic, Linq) are small battery driven diagnostic devices designed to continuously collect ECG information out of patients' bodies. Due to battery lifespan these devices have to be replaced every 2-4 years, whereas the clinically relevant monitoring time period may extend over decades of patients' life. Besides patients' discomfort replacement surgery is associated with medical risks and extensive utilization of health care resources.

As further example micromechanical system based implantable pressure sensors (e.g., Abbott, CardioMEMS) can only record intravascular pressure values once they are powered with external energy injection. For this the patient needs to lay on a transmitter pillow unit. This way the technology can only collect a snapshot picture of the biological information and the clinical patient scenario - obtained at the moment of transmission. Automatized continuous signal collection, storage and transmission is not possible. Energy management is the underlying system limitation here as well.

Due to the boundaries of today's energy management solutions combined sensors providing a multitude of biological signals for a holistic patient assessment are also not yet available.

Also in the application field of wildlife recharging or even replacement of the energy supply of a monitoring device applied to a wild animal is problematic, as the wild animal must firstly be trapped for such a procedure.

For this reason it would be of high value if the energy supply of a monitoring device could be designed in such a way that autonomous supply is made possible without the need for recharging or even replacement of the energy supply.

Therefore, it is the purpose of the invention to overcome the above-mentioned disadvantages of the state of the art and to provide a device to record, store and transmit signals and which is electrically self-sufficient and therefore does not need external charging or even battery driven replacement procedures.

Therefore, the present invention provides a device to record signals, which comprises
a multiple of microneedles (10) forming an array of microneedles;
a chip comprising at least one comparator with adaptive level, a sequence control circuit, at least one capacitor stack built by n capacitors and 2n switches, at least one buffer capacitor outside the at least one capacitor stack, at least two additional switches outside the at least one capacitor stack and a CMOS-Logic, wherein n E N, wherein the n capacitors are adapted to be sequentially charged by at least one microneedle of the array of microneedles, which functions as DC input source, one after the other and wherein the 2n switches of the capacitor stack couple the n capacitors selectively to at least one microneedle of the array of microneedles and wherein the buffer capacitor outside the at least one capacitor stack is dedicated to be charged from the n capacitors of the capacitor stack at once;
an interposer layer comprising holes for the multiple of microneedles;
a lid;
at least one coil;
at least one sensor;
wherein the chip, is located on one surface of the interposer layer;
wherein the lid and the interposer layer form a capsule for the chip and the at least one coil;
wherein the capsule carries an outer Far-Field electrode;
wherein each microneedle has a distal end which protrudes from the chip; and
wherein the device is adapted to be electrically self-sufficient due to harvesting of
electrical energy from an electrical energy source of the surrounding.

Further the invention provides a method collection signals utilizing a device according to the invention, wherein
- at least one microneedle of the array of microneedles is set to harvest energy and optionally to sense the amplitude of cellular electrical activity;
- energy is harvested from the surrounding and optionally the amplitude of cellular electrical activity is sensed at least by one microneedle;
- at least one sensor continuously records signals, which are stored and/or transferred to an external interrogation/programmer unit;
wherein harvested energy is used to operate the device.

### Detailed description

### Device configuration

The device according to the invention comprises a multiple of microneedles which form an array of microneedles. Every microneedle of the array of microneedles has a proximal and a distal end. In one embodiment of the invention, the microneedle according to the invention has a proximal end, which is shaped cylindrical with a diameter between 0.05 mm and 0.5 mm, preferably the proximal end has a diameter of 0.2 mm and a height between 0.05 mm and 0.5 mm, preferably with a height of 0.2 mm.

In a further embodiment of the invention the microneedle according to the invention has a proximal end which is shaped like a cuboid with a width and depth between 0.05 mm and 0.5 mm, preferably the width and depth of the cuboid is 0.2 mm. The height of the cuboid is between 0.05 mm and 0.5 mm, preferably the cuboid has a height of 0.2 mm.

From the proximal end, the microneedle comprises a tapered portion which connects a distal end with the proximal end. The distal end is needle shaped and has a length between 0.5 mm and 2.0 mm. The distal end of the microneedle is electrically conductive and shear stress resistant in the range of 5 to 50 Newton, which is comparable to the shear stress resistance of bonding wires. Preferably the microneedle is milled from one piece.

Preferably, the diameters of the distal ends of the multiple of microneedles are between 0,001 mm and 0.1 mm, preferably between 0.01 mm and 0.1 mm, most preferably the diameters of the distal ends of the multiple of microneedles are 0.02 mm. Thereby, the distal ends of the microneedles approximate cellular dimensions. The dimensions of the microneedles are therefore a lot smaller than any other electrodes in use today.

In a preferred embodiment of the invention each microneedle of the array of microneedles has the same shape and dimension.

In a preferred embodiment of the invention the device comprises between 5 and 10000 microneedles, preferably between 25 and 1000 microneedles, most preferably between 100 and 250 microneedles.

Principally the multiple of microneedles can be arranged on the chip in every way. In a preferred embodiment of the invention, the multiple of microneedles is arranged symmetrically to each other on the chip. Thereby, advantageously a largest possible number of microneedles can be arranged on the surface of the chip. Further, the regularity in the order of the microneedles simplifies production processes.

The microneedles comprise a material of the group comprising Platin/Iridium (Ptlr), gold, and fine metals. The material of the microneedles should be suitable for solder-connection with the chip or the interposer layer. In one embodiment of the invention, all materials comprised in the multiple of microneedles are bio-compatible and insensitive to body liquids. Bio-compatible in conjunction with the present invention means that no toxic interactions occur between the bio-compatible material and tissue, e.g. human tissue, animal tissue or plants.

Further, preferably, each microneedle is adapted to be able to harvest cellular energy. In one embodiment each microneedle is adapted to be able to harvest cellular energy and to sense intrinsic cellular activity. According to the invention, every microneedle of the multiple of microneedles is operable independent of the other microneedles. Accordingly, in one embodiment one microneedle could harvest energy while a neighboring microneedle is sensing intrinsic cellular electrical activity. The tasks of each microneedles can be redistributed at any time and thus adapted to the current requirements of the device.

Further, the device according to the invention comprises a chip and an interposer layer. In one embodiment of the invention the proximal end of each microneedle is soldered to the surface of the chip, which ensures that each microneedle of the array of a multiple of microneedles has a direct contact to the chip. In a further embodiment of the invention the proximal end of each microneedle is soldered to the surface of the interposer layer of the device. According to the invention each microneedle of the array of a multiple of microneedles is isolated from each other microneedle of the array of a multiple of microneedles. Further, the distal end of every microneedle protrudes from the chip and/or the interposer layer.

According to the invention the chip comprises all operations necessary to control the device's functions. Therefore, the chip comprises at least one comparator with adaptive level, a sequence control circuit, at least one capacitor stack built by n capacitors and 2n switches, at least one buffer capacitor outside the at least one capacitor stack, at least two additional switches outside the at least one capacitor stack and a CMOS-Logic, wherein n E N. Further, the chip comprises a digital memory such as SRAM, NVM or DRAM to store signals which are recorded by the at least one sensor.

According to the invention the device comprises an outer far-field electrode. The outer far field electrode is preferably positioned on top of the lid. The far-field electrode is adapted to derive an electrical signal between any microneedle of the arrays of microneedles and the far-field electrode and can function as potential reference point. By deriving an electrical signal between one of the microneedles of the array of microneedles and the far-field electrode, a larger spatial distance is created between the two derivation poles, compared, for example, to two adjacent microneedles of the array of microneedles. This can therefore be used to measure the electrical activity in a larger field.

This can be used for example to measure an ECG signal in a human or animal heart. Such an ECG recording "sees" more far-field signals and thus resembles more a classical surface ECG than a bipolar intracardiac lead by the microneedles of the array of microneedles. Accordingly, there is additional benefit in the interpretive content of such a far-field lead. With a device according to the invention implanted in the ventricle, the far-field signal can also be used to make statements about the electrical activity of the atria. Further, the far-field signal can help in the interpretation of cardiac arrhythmias. Life-threatening cardiac arrhythmias from the ventricle can be identified in the far-field ECG by changing the morphology of the ECG signal (for example morphology discrimination algorithms in ICD therapy). This is based on the fact that the electrical masses of larger activated cardiac muscle areas are included in the far-field ECG, which characteristically influence the shape and size of the signal. A strictly bipolar intracardiac near-field signal (e.g. between 2 microneedles of the array of microneedles) does not have this ability.

Advantageously, the device of the invention provides both information, on the one hand the far-field signal and on the other hand the signal sensed between two microneedles of the array of microneedles. Both information can be combined for interpreting the ECG signal.

### Self-sufficiency Energy harvesting

In a preferred embodiment of the invention the chip comprises at least one comparator with adaptive level, at least one capacitor stack built by n capacitors and 2n switches, at least one buffer capacitor outside the at least one capacitor stack, at least two additional switches outside the at least one capacitor stack for each needle of the array of microneedles.

According to the invention the 2n switches of the at least one capacitor stack couple the n capacitors selectively to at least one microneedle of the array of microneedles. Further, the n capacitors of the at least one capacitor stack are dedicated to be sequentially charged by at least one microneedle of the array of microneedles one after the other. And the at least one buffer capacitor outside the at least one capacitor stack is dedicated to be charged from the n capacitors of the capacitor stack at once.

Hence, the chip according to the invention comprises at least one capacitor stack, wherein the capacitor stack is built by n capacitors and 2n switches, wherein *n* ∈ *N*. The capacitor stack can comprise as much capacitors as can be accommodated constructively. In one embodiment of the invention n is between 2 and 20, more preferably between 2 and 14. The n capacitors of the capacitors stack are dedicated to be sequentially charged by at least one microneedle of the array of microneedles, which functions as DC input source, one after the other.

The 2n switches of the capacitor stack couple the n capacitors selectively to at least one microneedle of the array of microneedles in a way that every capacitor is sequentially charged by the DC input made available by the at least one microneedle of the array of microneedles one after the other. The DC input is made available since the microneedles couple directly electrically to an electrical energy source in the surrounding of the device.

In a preferred embodiment a suitable electrical energy source is a DC input source. Thereby, a suitable DC input has an electric potential of a few millivolts. Preferably the electric potential of the DC input source is between 1 mV and 1000 mV, more preferably between 1 mV and 500 mV, most preferably between 1 mV and 50 mV.

Suitable energy sources with electrical potentials in this range are for example bioelectric signals, radio signals, thermal sources or vibrations. The DC-input source can be intermittent, which means the DC-voltage is not always present. In case the energy source is a thermal source the thermal energy can be converted in electrical energy for example by a Peltier element. If the energy source are vibrations, the mechanical energy can be converted into electrical energy by motor. In a preferred embodiment of the invention the electrical energy source is selected from the group comprising bioelectric signals and radio signals. In one embodiment of the invention bioelectric signals from cell potential from living beings are used as DC input. Bioelectric signals of cells have typically an electric potential of 60-90mV and an internal resistance of 20 to 200 kOhm.

The small dimensions of the microneedles offer several advantages over the state of the art. Firstly, the redundancy and amplification of energy harvesting capabilities is achieved due to an exponential increase of electrically active harvesting units compared to current state of the art. In case the device is applied to a living being or a plant, microneedles according to the invention couple directly electrically to the cells of a living being or a plant. Thereby, e.g., one microneedle is in direct electrical contact with about 100 cells. Hence, the electrical signals of the microneedles according to the invention are near-field signals and are similar in form to the action potential of the cells of living beings. Further, microneedles according to the invention are able to sense cellular electrical activity and harvest energy directly from inside the tissue of a living being or plants due to their small dimensions.

Thermal sources can be for example the sun which is providing energy in the form of radiation, which could be collected. Further, temperature differences can be transformed into energy flows. In one embodiment artificial electromagnetic radiation, such as from radio transmissions can be transformed into energy. In this context, also biologically generated energy can be used. For example, the energy content of sugar, generated from photosynthesis, can be used to drive monitoring devices. Either by oxidizing the sugar, or indirectly through cell-energy transformation into muscular activity or electricity.

In a preferred embodiment of the invention the device is dedicated to collect small charges in the Nano-Coulomb-range and below.

The controlling and sequencing of the switches is generated from a usual CMOS-Logic, which is common to Microelectronics.

At least one buffer capacitor is situated outside the capacitor stack, which works as a buffer. According to the invention, the at least one buffer capacitor is dedicated to be charged from the n capacitors of the at least one capacitor stack at once. In a preferred embodiment of the invention, the chip comprises one buffer capacitor outside the capacitor stack. In a further preferred embodiment of the invention the chip comprises two buffer capacitors outside the capacitor stack.

Furthermore, the chip comprises at least two additional switches outside the capacitor stack. In a preferred embodiment of the invention the chip comprises two additional switches outside the capacitor stack. The additional switches are dedicated to selectively couple the capacitor stack to the at least one buffer capacitor outside the capacitor stack or to a further optional capacitor stack.

In a further preferred embodiment, the chip comprises four additional switches outside the capacitor stack. Preferably the chip comprises four additional switches outside the at least one capacitor stack if the chip comprises a first buffer capacitor outside the at least one capacitor stack and a second buffer capacitor outside the at least one capacitor stack. In this embodiment two additional switches are dedicated to selectively connect the at least one capacitor stack to the first buffer capacitor outside the capacitor stack and the two further additional switches are dedicated to selectively connect the at least one capacitor stack to the second buffer capacitor outside the capacitor stack.

Accordingly, in one preferred embodiment the chip according to the invention comprises two buffer capacitors outside the capacitor stack as buffer capacitors outside the at least one capacitor stack and four additional switch outside the at least one capacitor stack.

From its physical construction as a stack, the n capacitors of the capacitor stack are all connected in series electrically. Furthermore, in one embodiment of the invention, the at least one capacitor stack comprises at least three conductive plates wherein the conductive plates have a top-side and a bottom-side and wherein the top-side of at least one conductive plate is part of a first capacitor and the bottom-side of the at least one conductive plate is part of a neighboring further capacitor. Furthermore, the capacitor stack comprises an isolating material between the conductive plates in a way that a capacitor is built.

In a preferred embodiment of the invention, a capacitor stack with n capacitors comprises m=n+1 conductive plates. According to the invention the first conductor n=1 is built between the bottom-side of the first conductive plate (m=1) and the top-side of the second conductive plate (m=2). The neighboring conductor (n=2) is built between the bottom-side of the second conductive plate (m=2) and the top-side of the third conductive plate (m=3) and so on.

The capacitance of the capacitors built according to the invention is quite wide ranging from 1 nF down to 1fF and even below. It depends on plate geometries and the dielectric material employed between the plates. Typical dielectric materials are SiO2 or plastic, but other dielectric materials are possible.

The arrangement of the conductors in a capacitor stack with n capacitors according to the invention has the advantage that the inner conductive plates, which means plates m=2 to m=n form no or just very small parasitic capacitances to the outside of the stack. Parasitic capacitances are well known in the art. They arise at the interfaces of capacitors to the surrounding and are unwanted as those have to be charged at every charge cycle of the capacitor. This process lowers the charging efficiency of the capacitor and therefore its end-charging voltage. Accordingly, in the state of the art every capacitor has two interfaces to the surrounding and therefore two interfaces where parasitic capacitances arise.

The capacitor stack according to the invention is able to provide n capacitors, wherein only the first and the last capacitor have a substantial interface to the surrounding. Therefore, advantageously, only at these two interfaces parasitic capacitances will form. Accordingly, the charging efficiency of the n capacitors of the capacitor stack is increased as well as the end-charging voltage.

Furthermore, in a preferred embodiment of the invention, all capacitors are connected in series electrically.

In one preferred embodiment of the invention the at least one capacitor stack built by n capacitors and 2n switches, the at least one buffer capacitor outside the at least one capacitor stack and the at least two additional switches outside the at least one capacitor stack are configured as an integrated circuit wherein switches are realized as transistors and capacitors are realized by conductive plates from integrated circuit technology.

Preferably the conductive plates are made of material selected from the group comprising metal or polysilicon or any other conductive material from integrated circuit technology. Suitable metals are copper and aluminum and tungsten.

In one embodiment of the invention the isolating material is selected from the group comprising SiO₂, SiN and Hf₂O and stacks thereof.

As described above the capacitor stack is internally nearly perfect if it comes to storing the applied charges, as the field is nicely confined internally. Unfortunately at the first and last conductive plates still some parasitic capacitances will form. In view not to lose the energy stored in those external parasitic capacitances, according to the invention, an inductor can be applied to perform intermediate storage in a resonant circuit configuration.

Accordingly, in one embodiment of the invention the chip comprises additionally an inductor. Preferably small inductors are integrated monolithically in the integrated circuit. According to the invention the switching frequency is chosen high enough so that the resonant frequency of the parasitic capacitor and the inductivity equals the inverse of the total charging/discharging cycle time of the capacitor stack. In addition the charging/discharging timing of the capacitor stack should be adapted such that a sine-curve is approximated.

Practical inductivity values in integrated circuits will be in the range 1-10 µH when 100 windings will wrap around a typical chip of 25 mm² size. Parasitic capacitor values will range between 1-10 pF for a typical capacitor stack. For this setting, the resonance frequencies will be found between 10-200 MHz. The charging frequencies of the capacitances in the capacitor stack in consequence will have to be 2n higher.

In another preferred embodiment the chip comprises several capacitor stacks wherein every capacitor stack is dedicated to charge another capacitor stack and one capacitor stack is dedicated to charge at least one buffer capacitor outsides the capacitor stacks. Thereby, cascading of the sequential small charge collection according to the invention is possible.

Several capacitor stacks are preferably connected by switches outside the capacitor stacks, most preferably always two capacitor stacks are connected by two switches outside the capacitor stacks. In one embodiment of the invention the device comprises x capacitor stacks and 2x switches outside the capacitor stacks, wherein *x* ∈ *N*. In one embodiment of the invention the device comprises 1 to 20 capacitor stacks, preferably 5 to 15, most preferably 13 to 15, as this is within the capabilities of current semiconductor production technologies.

However, the charging frequency of a further capacitor stack is n-times slower than the charging frequency of the first capacitor stack (with n being the number of capacitors in the first capacitor stack). In principle the n capacitors of the first capacitor stack are charged by the DC input of at least one microneedle of the array of microneedles one after the other. Afterwards the n capacitors of the first capacitor stack are discharged at once to one capacitor of a further capacitor stack. In case the further capacitor stack is built by k capacitors, k charging cycles are needed to charge the k capacitors of the further capacitor stack one after the other, wherein *k* E N. If all capacitors of the further capacitor stack are charged they are discharged to a further capacitor outside the capacitor stack at once. In total, the entire discharge occurs at a frequency k · n lower than the charging frequency of the first capacitor stack. The maximum voltage of the second stack is k · n the feeding voltage of the DC input source. For example with 10 mV at the at least one microneedle of the array of microneedles, and 10 capacitors on each capacitors stack, 1 V can be realized as output at maximum.

According to the invention, every further capacitor stack is dedicated to be fed by positive or negative voltages from another capacitor stack. Therefore, the switches outside the capacitor stacks connecting the capacitor stacks have to be sequenced accordingly. If the first capacitor stack provides positive or negative charge, charging of the second capacitor stack has to be done accordingly.

In an embodiment of the invention the sequencing of the switches is generated from a usual CMOS-Logic, which is common to Microelectronics. For the CMOS-logic to function, voltages of a few hundred millivolts are required. Typical state of the art semiconductor technology operates at around 1 Volt or slightly below. Since the device according to the invention collects energy starting with a few millivolts at the source, this voltage is too low to operate the CMOS-logic.

In one embodiment of the invention the device comprises at least one further capacitor. The at least one further capacitor can be comprised on the chip or can be located on the interposer layer outside the chip. The at least one further capacitor of the device serves preferably as buffer capacitor for all microneedles of the array of microneedles and the capacitor stacks assigned to them. Furthermore, the at least one further capacitor of the device can serve for energy transfer from an external energy source, e.g. for the startup process. In one embodiment the at least one further capacitor is connected to the chip by the interface for power management comprised on the chip.

### Startup circuit

However, after collection and cascading, voltages in the 1-Volt domain can be obtained, which is enough to operate the CMOS-logic. For this reason, a startup circuit is required, to make sure the logic can be powered and the switches are operated to perform energy collection from the tiny sources.

For this, a magnetic coupling over coils is proposed. The outer coil is excited with alternate current, creating a magnetic alternating field. Through this field the startup energy is transmitted to the coil on the integrated circuit, which recuperates the startup energy.

According to the invention the device comprises at least one coil. Preferably the coil is dedicated to receiving a startup energy by magnetic coupling with another coil and to transmit data in and out of the device. In one embodiment at least one coil is located on the interposer layer. In a further embodiment of the invention at least one coil is part of the chip in the sense that the at least one coil is wound around the chip. In a further embodiment of the invention the device contains two external wired loops forming an external signal transmitter coil. In this embodiment the total diameter of the coil is between 1 and 2 cm. However, in each of these embodiments the chip comprises an interface for power management to connect the at least one coil of the device to the CMOS-logic comprised on the chip.

### Interposer layer

Further the device according to the invention comprises an interposer layer and a lid.

The interposer layer serves as assembly platform for the chip and comprises a material of the group comprising FR4 materials, epoxy-resin, Poly(methyl methacrylate) (PMMA), ceramics, silicon, silicon dioxide (SiO₂), glass and plastics. FR4 materials are a class of flame resistant composite materials comprising woven fiberglass and epoxy resin. Principally, the materials comprised in the interposer layer have to be non-electrically conductive. In one embodiment of the invention all materials comprised in the interposer layer are bio-compatible and insensitive to body liquids.

In one embodiment of the invention the interposer layer comprises holes, each hole being suitable for the distal end of a microneedle to pass through. According to the invention the interposer layer comprises a hole for each microneedle of the array of microneedles. Thus, in a preferred embodiment the interposer layer comprises as many holes as the array of a multiple of microneedles comprises microneedles. In this embodiment the microneedles are soldered to a surface of the chip.

The chip is positioned on top of the interposer layer and the microneedles of the array of a multiple of microneedles pass through the holes in the interposer layer. Hence, every microneedle of the array of microneedles passes through a separate hole in the interposer layer. Advantageously, the holes in the interposer layer are arranged in a way that all microneedles of the array of a multiple of microneedles which are soldered to the chip can pass through without making contact to the interposer layer.

Every hole in the interposer layer with a microneedle passing through is sealed to the surrounding with a non- conductive material. Suitable non-conductive materials are for example epoxy-resin, Poly(methyl methacrylate) (PMMA), glass and plastics. Thereby, no fluids from the environment can penetrate to the chip through the holes.

In a further embodiment each microneedle of the array of microneedles is soldered to the interposer layer. In this embodiment the interposer layer comprises a wiring connecting each microneedle of the array of microneedles to the chip, thereby connecting each microneedle of the array of microneedles to at least one capacitor stack.

The device comprises at least one sensor and in one embodiment the device further comprises at least one further capacitor. In this embodiment the interposer layer comprises a wiring connecting the chip, the at least one senor and the at least one further capacitor with each other.

Further, in one embodiment the interposer layer serves as assembly platform for the at least one further capacitor and/or the at least one coil of the device. In this embodiment the at least one further capacitor and/or the at least one coil are preferably positioned next to the chip on the interposer layer.

According to the invention the device further comprises a lid. The lid covers the chip from the surrounding, wherein the lid is sealed to the interposer layer. Sealing can be done by adhesives or soldering tin. If adhesives are used the adhesive should be hardened. In one embodiment of the invention the sealing is bio-compatible and insensitive to body fluids. Accordingly, the lid and the interposer layer form a capsule for the chip. The lid and the interposer layer shield the electronic parts from surrounding, e.g. from body-fluids like blood by forming a capsule.

In one embodiment of the invention next to the chip on top of the interposer layer at least one sensor and/or at least one capacitor and/or at least one coil are positioned. The chip, the at least one sensor and/or the at least one capacitor and/or the at least one coil are located on one surface of the interposer layer and the lid covers the chip, the at least one sensor and/or the at least one capacitor and/or the at least one coil from the surrounding, wherein the lid is sealed to the interposer layer, as already described. Thereby, the lid and the interposer layer form a capsule for the chip, the at least one sensor and/or the at least one capacitor and/or the at least one coil. Preferably, the lid and the interposer layer shield all electronic parts comprised in the device from the surrounding body-fluids like blood, e.g. from body fluids by forming a capsule.

The lid comprises a material of the group comprising comprising FR4 materials, epoxy-resin, Poly(methyl methacrylate) (PMMA), ceramics, silicon, silicon dioxide (SiO₂), glass, plastics and metals. Suitable metals are for example aluminum, or aluminum vaporized with tungsten. According to the invention all materials comprised in the lid are bio-compatible and insensitive to body liquids. In one embodiment of the invention the lid comprises a translucent material, thereby light can reach a light sensitive sensor positioned on the chip under the lid.

In one embodiment of the invention fixation of the device to a surrounding surface is obtained through an adhesive. A suitable adhesive should be chosen in dependence of the type of material the device should be fixed to. In one embodiment the adhesive is a biological adhesive (e.g. fibrin-based materials), which is therefore bio-compatible and insensitive to body fluids.

In one further embodiment of the invention the interposer layer further comprises at least two fixing holes outside the lid. Advantageously, a fixing hole is positioned outside the lid on each side of the lid in the interposer layer. The fixing holes are suited to serve for the fixation of the device into human tissue. The device can be fixated by screws, clamps or such like devices through the fixing holes in the interposer layer outside the lid.

The device according to the invention is between 1 mm and 5 cm long, between 1 mm and 10 mm wide and between 3 mm and 10 mm high.

Advantageously, the device according to the invention is adapted to be electrically self-sufficient.

According to the invention, the chip comprises a sequence control circuit. This circuit controls the functionality of the device and determines the workflow of the functions of the device. All interfaces comprised on the chip like I/O-interface, sensor interface and interface for power management are interfaces to the sequence control circuit.

### Sensor

According to the invention the device comprises at least one sensor. The at least one sensor is selected from the group comprising electrical pin-sensors, electrical far-field sensors, micromechanical (MEMS) activity sensors, electrostatical accelerometers, piezoceramic accelerometers, micromechanical (MEMS) pressure sensors, micromechanical (MEMS) temperature sensors, light source-based sensors, microneedles.

According to the intervention the at least one sensor is embedded on and within the capsule in addition to the chip. The at least one sensor can be comprised on the chip or can be located on the interposer layer outside the chip.

In one embodiment the device comprises more than one sensor. The device can comprise between 1 and 5 sensors, preferably between 1 and 4 sensors, more preferably between 1 and 3 sensors. All sensors are embedded on and within the capsule.

In one embodiment the device is used to monitor signals from living beings. In a preferred embodiment of the invention the sensor is a sensor for
(i) electrograms obtained from between at least two microneedles within human tissue
(ii) Far-Field electrical signals obtained from between one microneedle and an electrode on the outer device capsule,
(iii) motion information obtained from embedded activity sensors such as a micromechanical (MEMS) activity sensor, an electrostatical accelerometer, or a piezoceramic accelerometer,
(iv) pressure information obtained from embedded pressure sensors such as a micromechanical (MEMS) pressure sensor,
(v) temperature information obtained from embedded temperature sensors such as a micromechanical (MEMS) temperature sensor,
(vi) oxygen levels obtained embedded light source-based sensor technology,
(vii) sugar levels obtained embedded light source-based sensor technology.

The device comprises a sensor interface to connect the sensor with the chip.

In one embodiment at least one microneedle of the array of microneedles functions as sensor. This is preferably the case if the device is used to monitor parameters in a living being. According to this embodiment at least one microneedle of the array of microneedles is suited to sense cellular electrical activitiy.

Sensing of cellular electrical activity requires measurement of the amplitude of the actual potential with regards to a reference level or ground. Generally, this is performed with a comparator circuit. The measurement of the tissue potential is standard and in use with current cardiac pacemakers, neuromodulators and others.

According to the invention, the chip comprises at least one comparator with adaptive reference level. In a preferred embodiment of the invention the chip comprises a comparator with adaptive reference level for each microneedle of the array of microneedles, wherein each microneedle of the array of microneedles is electrically connected to one comparator circuit on the chip. In one embodiment the comparator can be an A/D converter.

According to the invention, every microneedle of the array of microneedles is adapted to be able to sense the amplitude of the intrinsic cellular electrical activity by the standard procedure of measurement of tissue potential. Advantageously, these measurements can be performed on one microneedle of the array of microneedles, on selected microneedles of the array of microneedles or on all of the microneedles of the array of microneedles. The redundancy of microneedles in the array of microneedles provides a number of beneficial features. How many and which of the microneedles of the array of microneedles perform sensing is programmed via the external programmer unit.

In one embodiment the cellular electrical activity is sensed continuously. In a further embodiment of the invention the cellular electrical activity is sensed in a way that it is monitored when the amplitude of the cellular electrical activity exceeds a reference level, when the amplitude of the cellular electrical activity falls below the reference level and what the maximum amplitude of the cellular electrical activity during a cellular cycle is. Thereby, timing points of the individual progression are monitored. Further, the amplitude of the cellular electrical activity is sensed by at least one microneedle of the array of microneedles and recorded by the external programmer unit or in one embodiment by an internal data memory device, which is comprised in the device. In case the amplitude of the cellular electrical activity is recorded by an internal data memory device, the chip according to the invention further comprises a suited internal memory device.

Further, the amplitude of the actual tissue potential sensed by the microneedles of the array of microneedles selected for sensing of cellular electrical activity is compared to a reference level by the at least one comparator. Principally the reference level for each comparator is programmable. In one embodiment of the invention a reference level is programmed individually for each microneedle of the array of microneedles. In a further embodiment of the invention, the reference level is the same for each comparator comprised on the chip. In one embodiment of the invention the reference level of the at least one comparator is programmable between 0.1 mV and 10.0 mV.

Hence, the reference level can be the same for all microneedles of the array of microneedles sensing the cellular electrical activity, but can also be different for each microneedle of the array of microneedles. By programming the reference level of each comparator individually, optimized timing points for all microneedles of the array of microneedles which are penetrating the tissue at different positions can be provided. This is very advantageously, since the timing of all functions of the device according to the invention can be programmed according to individual clinical scenario. This increases on the one hand safety aspects, since the cellular electrical activity is sensed by several individual microneedles of the array of microneedles, which can be understood as sensing with several individual electrodes. If one microneedle of the array of microneedles does not function correctly another microneedle can be programmed to perform sensing function. Accordingly, sensing function is provided in a redundantly way. In contrast, in state-of-the-art electrogram recording devices only one electrode is provided for sensing cellular electrical activity. If this electrode does not function correctly, the device has to be replaced. Hence, the device according to the invention reduces the risk of new operations.

On the other hand, in one embodiment of the invention the amplitudes of the cellular electrical activity sensed by several microneedles of the array of microneedles are compared by the external programmer unit and/or by the sequence control circuit. Hence, electronic malfunctions/misinterpretations of the electrical signal become less likely because permanently the signals of several microneedles of the array of microneedles are compared electronically.

Further, the efficiency of the device is increased compared to state of the art, since the microneedles of the array of microneedles used for performing energy harvesting and/or sensing of cellular electrical energy can be selected depending on faultless function of the respective microneedles of the array of microneedles.

Due to the multiple microneedles sensing the cellular electrical energy independent from each other and which sense temporally and spatial aligned, the typical vulnerability of state-of-the-art bio-signal collection to electrical signal misinterpretation due to external electrical noise or electrical far-field signals is diminished.

### Harvesting

Harvesting is done by a method comprising at least one capacitor stack build by n capacitors and 2n switches, at least one buffer capacitor outside the capacitor stack, at least two additional switches and at least one microneedle of the array of microneedles as DC input source, comprising the steps
a. the n capacitors of the capacitor stack are sequentially charged by coupling one capacitor after the other to at least one microneedle of the array of microneedles by selectively closing the switches;
b. discharging the n capacitors of the capacitor stack into the at least one buffer capacitor outside the capacitor stack;
wherein the at least one microneedle of the array of microneedles couples directly electrically to the surrounding which thereby functioning as DC input source. Steps a. and b. define one harvesting cycle.

Suitable DC input sources of the surrounding are selected from the group comprising bioelectric signals, radio signals, thermal sources or vibrations. In one embodiment of the invention bioelectric signals from cell potential from living beings are used as DC input.

In a preferred embodiment of the method of the invention the n capacitors of the capacitor stack are sequentially charged one after the other in n charging cycles and the n capacitors of the capacitor stack are discharged in an n+1^{st} cycle into at least one buffer capacitor outside the capacitor stack at once.

Fundamentally, the capacitors of a capacitor stack could be charged in any order. However, in order to reduce the recharging of the parasitic capacitances which arise at the interfaces to the surrounding, the following charging scheme is proposed. According to a preferred embodiment of the invention, the n capacitors of the capacitor stack are sequentially charged one after the other in n charging cycles, wherein the first capacitor is charged, afterwards the capacitor which is next to the first one is charged, afterwards the capacitor which is next to the one charged before is charged until all n capacitors are charged.

If all capacitors of a capacitor stack are charged, the capacitors of the capacitor stack are all discharged into at least one buffer capacitor outside the capacitor stack at once. This is done by selectively closing the switches of the capacitor stack and the switches outside the capacitor stack.

In a further embodiment of the invention a bipolar charging of the capacitor stack can be done. Fundamentally, each capacitor of a capacitor stack can be charged to positive or negative voltages, depending which plate of the capacitor is grounded. As already described, capacitors in the capacitor stack are being loaded sequentially. While one plate is grounded, the other plate is charged to a fraction of the input voltage. Which means, that capacitors in the capacitor stack above the currently grounded plate are pushed to positive voltages, whereas the plates below the currently grounded plate are pushed to negative voltages. Accordingly, the capacitors of the capacitor stack can be charged to positive or negative voltages, by closing the switches inside the capacitor stack in an appropriate manner, thereby selecting the grounded plate of the each capacitor in the capacitor stack.

If bipolar charging of the capacitor stack is done, preferably two buffer capacitors outside the capacitors stack are used as buffer capacitors. In this embodiment of the invention the at least one capacitor stack is first charged with positive voltages and after all capacitors in the capacitor stack are charged, all capacitors of the capacitor stack are discharged into the first buffer capacitor outside the capacitor stack. Afterwards the capacitors of the capacitor stack are charged with negative voltages and after all capacitors in the capacitor stack are charged all capacitors of the capacitor stack are discharged into the second buffer capacitor outside the capacitor stack.

Since parasitic capacitances which arise at the interfaces to the surrounding have also to be charged at each charging procedure, it is most advantageous to always charge neighboring capacitor and not to "jump around" between the capacitors in the capacitor stack. Therefore, if bipolar charging of the capacitor stack is done the capacitors of the capacitor stack are sequentially charged the n capacitors are discharged into a first buffer capacitor outside the capacitor stack, afterwards the n capacitors of the capacitor stack are sequentially charged in the reversed order and after the n capacitors are charged the n capacitors are discharged into a second buffer capacitor outside the capacitor stack.

Accordingly, in a preferred embodiment of the invention after the n capacitors of the capacitor stack are sequentially charged the n capacitors are discharged into a first buffer capacitor outside the capacitor stack, afterwards the n capacitors of the capacitor stack are sequentially charged in the reversed order and after the n capacitors are charged the n capacitors are discharged into a second buffer capacitor outside the capacitor stack.

According to the invention the n capacitors of a capacitor stack can be discharged at once into one capacitor of a further capacitor stack.

The charging sequence for the second capacitor stack is derived from the first stack and couples to its timing. Instead of discharging the first capacitor stack into a buffer capacitance, it is discharged into one of the capacitors forming the second capacitor stack. Fundamentally it could be any of capacitors of the second capacitor stack, but practically the charging of the second capacitor stack should follow the same method already described. Which means charging of the capacitors of a capacitor stack should be done by charging neighboring capacitors. As the second capacitor stack is also loaded with some parasitic capacitance to the outside, sequentially charging the second stack as described will keep the charge flown into the parasitic capacitances to a minimum at each step.

One of the embodiments of the discharge circuit is a bipolar setting. This allows the charging of the second capacitor stack with negative and positive charge depending on the sequence. When the negative charge is transferred to the second capacitor stack, care has to be taken that the transistor switches are operated such that the charge on the stack is added with reverse polarity, so that this charge is accumulated on the second stack and not subtracted.

Parallelizing the sequential charge collection according to the invention, energy collection is multiplied and therefore the output-power of the device is increased.

In one embodiment of the invention the device comprises at least one further capacitor. The further capacitor can serve as buffer capacitor for the harvested energy or for energy which is transferred from an external energy source.

According to the invention energy harvesting can be done on one microneedle of the array of microneedles, on several microneedles of the array of microneedles or on all of the microneedles of the array of microneedles simultaneously.

Furthermore, in one embodiment of the invention a selection mechanism is implemented to select high yielding microneedles of the array of microneedles for energy harvesting and to discard low yielding microneedles of the array of microneedles. In one embodiment of the invention it is programmed which microneedles of the array of microneedles are utilized for energy harvesting. This is done via the external programmer unit. In a further embodiment of the invention the microneedles of the array of microneedles which are utilized for energy harvesting are selected due to the cellular electrical energy sensed by the individual microneedles of the array of microneedles. This is regulated internally by the sequence control circuit on the chip, advantageously no regulation by the external programmer unit is necessary. Thereby, the microneedles of the array of microneedles are selected for energy harvesting that achieve the highest energy yield.

In one embodiment of the invention the device harvests energy from tissue cells. In this embodiment every microneedle of the array of microneedles is adapted to harvest cellular energy. Human, animal organs or plants for example consist of billions of cells. E.g., the left main chamber of the human heart (left ventricle) contains a total of approximately 6 billion cells. Each cell acts as a battery which is discharged and charged once during each cellular cycle. That function is mediated by the exchange of Sodium and Potassium through ion channels in the cellular membrane. The actual electrical energy turnover of an individual cell is small, however, harvesting from multiple cells and multiple times can collect a significant amount of electrical energy.

Even if the transmembrane voltages cannot be directly accessed, as the microneedles of the array of microneedles are too large to reach an individual intracellular space, portions of the produced cellular electrical energy are collectible from the outside intercellular space. As one microneedle of the array of microneedles touches a sequence of cells (approximately 100 cells) which operate synchronously, the collectable energy increases. The same holds true if the device according to the invention is inserted into animal tissue or a plant.

In this embodiment, sensing of the amplitude of the cellular electrical activity and energy harvesting happen together on a microneedle. If a cellular electrical activity above the reference level is detected, harvesting of electrical energy starts. In one embodiment of the invention, harvesting is carried out with repeated charging cycles until the cellular electrical activity falls below the reference level. Which means the microneedle harvests cellular electrical energy multiple times throughout a singular cellular cycle. In this embodiment the time interval for energy harvesting depends on the depolarization rate and is approximately between 150 ms and 300 ms. In a further embodiment of the invention harvesting is carried out until a programmable time-out is reached. In this case the programmable time-out could be between 200 ms and 300 ms.

In one embodiment of the invention the device is adapted to perform several harvesting cycles during a singular cellular cycle in order to additionally optimize the harvested energy amount.

No matter which DC input source is utilized the device of the invention is adapted to be electrically self-sufficient due to harvesting of electrical energy from the surrounding. Since the harvested energy is uses to operate all functions of the device, the device of the present invention is not dependent on a power supply from a battery, which means the device according to the invention is electrically self-sufficient, which means electrically autonomous. The energy required for monitoring in the sense of the invention, is provided out of the environment into which the monitoring device according to the invention is embedded. All disadvantages associated with battery operation are eliminated. Hence, there is no need for any kind of recharge procedure for a battery or even a whole replacement of a device by a new one due to an empty battery.

### Programmability

Further, the chip of the device according to the invention is adapted to communicate with an external interrogation/programmer unit. External means that the unit must not be in direct contact with the chip at all. The communication is preferably done via externally applied electromagnetic fields. Accordingly, in one embodiment the device further comprises at least one coil to communicate with the external interrogation/programmer unit, thereby the coil functions as receiver and transmitter. Furthermore, the chip comprises an I/O interface for data transmission from the external interrogation/programmer unit via the coil of the device to the chip. This has the advantage, that the functionality of the device can be proofed and data recorded by the at least one sensor can be transmitted, surveyed and evaluated. Although, adjustments in the functionality of the device are possible without physical contact to the device.

Accordingly in a preferred embodiment of the invention the device further comprises an external interrogation/programmer unit. The external interrogation/programmer unit is selected from a group comprising tablets, smartphones and PC's. The external interrogation/programmer unit is adapted to communicate with the chip, therefore in a preferred embodiment a coil for transmitting and receiving is comprised in the external interrogation/programmer unit.

Accordingly in one embodiment of the invention the device comprises at least two coils, wherein one coil is adapted to receive a startup energy for the CMOS-logic and one coil is adapted to function as receiver and transmitter to the external programmer unit. Both coils can be located on the interposer layer or can wound around the chip as already described. In another embodiment one coil can be located on the interposer layer and another coil can be wound around the chip. In another embodiment one coil can be located externally of the capsule attached to the interposer in a wired loop design.

Advantageously, in a preferred embodiment the coil which is comprised in/at the device and used to communicate with the external programmer unit and the coil which is comprised in/at the device and used to receive a startup energy for the CMOS-Logic are the same coil.

Further the invention comprises a method for collection signals utilizing a device according to the invention, characterized in that
- at least one microneedle of the array of microneedles is set to harvest energy and optionally at least one microneedle of the array of microneedles is set to sense the amplitude of cellular electrical activity;
- energy is harvested and optionally the amplitude of cellular electrical activity is sensed at least by one microneedle;
- at least one sensor continuously records signals, which are stored and/or transferred to an external interrogation/programmer unit;
wherein harvested energy is used to operate the device.

All features described for the device of the invention apply likewise to the method of the invention and vice versa.

According to the method of the invention at least one of the microneedles of the array of microneedles is set to harvest energy. Optionally at least one microneedle of the array of microneedles is set to sense the amplitude of cellular electrical activity. The latter is preferably done if the device is inserted into the tissue of a living being. In this case preferably the at least one microneedle of the array of microneedles set to harvest energy is also set to sense the amplitude of cellular electrical activity. This is done via the external programmer unit. Advantageously, adjustments of the device can be done any time via the external programmer unit.

The device is positioned in a surrounding environment in a way that the microneedles of the array of microneedles are in direct contact to a suitable DC input source of the surrounding. In case radio signals or thermal sources are used as DC input source, the microneedle of the array of microneedles protrude into the air surrounding the device. If bioelectrical signals such as cellular electrical activity is used as DC input source the microneedles of the array of microneedles can be inserted into plant tissue and energy is harvested from tissue cells.

According to the invention energy is harvested by at least one microneedle of the array of microneedles. The harvested energy is used to operate the device of the invention. Associated advantages have already been sufficiently explained.

Further, at least one sensor continuously records signals, which are stored and/or transferred to an external interrogation/programmer unit. In one embodiment of the invention more than one sensor continuously records signals. In this case advantageously more than one parameter can be recorded and monitored.

In one embodiment of the invention a signal recorded by the at least one sensor can after analog-to-digital conversion be stored in digital memories such as SRAM. In a further embodiment of the invention the signals recorded by the at least one sensor are transferred to an external interrogation/programmer unit. Accordingly, data evaluation and therefore interpreting of the signal or of combinations of signals can be done by the external interrogation/programmer unit.

Optionally the amplitude of cellular electrical activity is sensed at least by one microneedle of the array of microneedles. This is preferably done in case the device is applied into tissue of a living being and harvests cellular electrical activity. Therefore in one embodiment the method of the invention comprises the following steps:
- the microneedles (10) of the array of microneedles (10) are inserted into tissue of a living being;
- at least one reference level for the cellular electrical activity is set;
- at least one microneedle (10) of the array of microneedles (10) is set to sense the amplitude of the cellular electrical activity and to harvest energy;
- the amplitude of the cellular electrical activity is sensed by at least by one microneedle and energy is harvested if the amplitude of the cellular electrical activity is above the at least one reference level by at least by one microneedle;
- at least one sensor continuously records signals, which are stored and/or transferred to an external interrogation/programmer unit;
wherein the harvested energy is used to operate the device.

Accordingly a reference level for the cellular electrical activity is set by the external programmer unit. Further, at least one microneedle of the array of microneedles is set to sense cellular electrical activity and to harvest energy. This is also done via the external programmer unit. These steps can be done immediately after the implantation of the device into the tissue. Advantageously, adjustments of these parameters can be done any time via the external programmer unit.

According to the method, the amplitude of the cellular electrical energy is sensed and energy is harvested at least by one microneedle of the array of microneedles. The harvested energy is collected into the at least one buffer capacitor. In one embodiment of the invention the harvested energy is collected into multiple buffer capacitors, wherein the multiple buffer capacitors constitute a buffer capacitors-array. According to the invention the amplitude of the cellular electrical activity is sensed by at least by one microneedle and energy is harvested if the amplitude of the cellular electrical activity is above the at least one reference level by at least by one microneedle.

The method and the device of the invention can be used to monitor biological signals from living beings or plants, environmental parameters such as temperature, pressure, geometric coordinates (location), concentration of chemical substances e.g. oxygen, CO₂, Nitrogen, or hazardous substances like CO, CH₄, NH₃ and such like, electrical, magnetic or electromagnetic fields in strength and orientation, motion.

In one embodiment of the invention the method and the device can be used for recording biological signals encompass electrograms obtained from at least one microneedle within human tissue, biological signals encompass Far-Field electrical signals obtained from an electrode on the outer device capsule, organ and body motion information obtained from embedded activity sensors, intracavity and intravascular pressure information obtained from embedded pressure sensors, body temperature information obtained from embedded temperature sensors, blood oxygen level obtained from oxygen level sensors, blood sugar level obtained from sugar level sensors.

The invention is further described by 6 figures and 3 examples.
- Figure 1: illustrates one embodiment of the device according to the invention in side view;
- Figure 2: illustrates one embodiment of the device according to the invention in top view;
- Figure 3: illustrates one further embodiment of the device according to the invention in top view;
- Figure 4: illustrates one embodiment of a microneedle of the array of microneedles;
- Figure 5: illustrates an embodiment of the device;
- Figure 6: illustrates harvesting during an event of electrical cell activity.

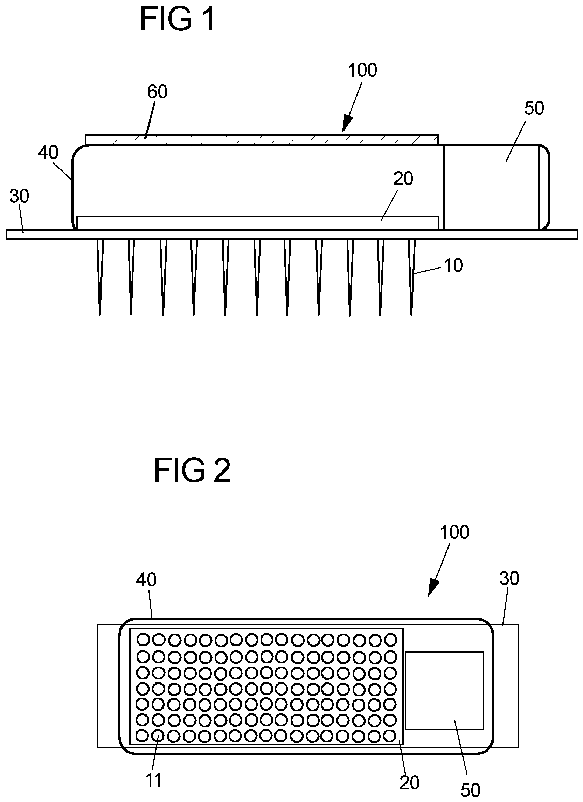

**Figure 1** illustrates one embodiment of the device 100 according to the invention in side view. On top of the interposer layer 30 the chip 20, a sensor 50 are positioned. The sensor 50 is a MEMS-pressure sensor and touches the lid 40, thereby the pressure transducer membrane is in contact with the surrounding. Furthermore, a coil is positioned on top of the interposer layer 30, which is not shown in the figure for the sake of clarity. The chip 20, the sensor 50 and the coil are covered by lid 40. The lid 40 covers the chip 20 and the sensor 50 from the surrounding, wherein the lid 40 is sealed to the interposer layer 30. Sealing can be done by adhesives or soldering tin. If adhesives are used the adhesive should be hardened. Accordingly, the lid 40 and the interposer layer 30 form a capsule for the chip 20 and the sensor 50. The lid 40 and the interposer-layer 30 shield the electronic parts from surrounding e.g. from body fluids by forming a capsule. The proximal end of each microneedle 10 is soldered to the surface of the chip 20, which ensures that each microneedle 10 of the array of a multiple of microneedles has a direct contact to the chip 20. According to the invention each microneedle 10 of the array of a multiple of microneedles is isolated from each other microneedle 10 of the array of a multiple of microneedles. Further, the distal end of every microneedle 10 protrudes from the chip 20 and the interposer layer 30. On top of the lid 40 the far-file electrode 60 is positioned.

**Figure 2** illustrates one embodiment of the device 100 according to the invention in top view. Again, the chip 20, the sensor 50 and the coil are positioned on the interposer layer 30 and covered by the lid 40. The proximal end of each microneedle 10 is soldered by soldering points 11 to the surface of the chip 20, which ensures that each microneedle 10 of the array of a multiple of microneedles has a direct contact to the chip 20. The outer-far field electrode 60 is not drawn to enable a top view on the chip with the soldering points 11.

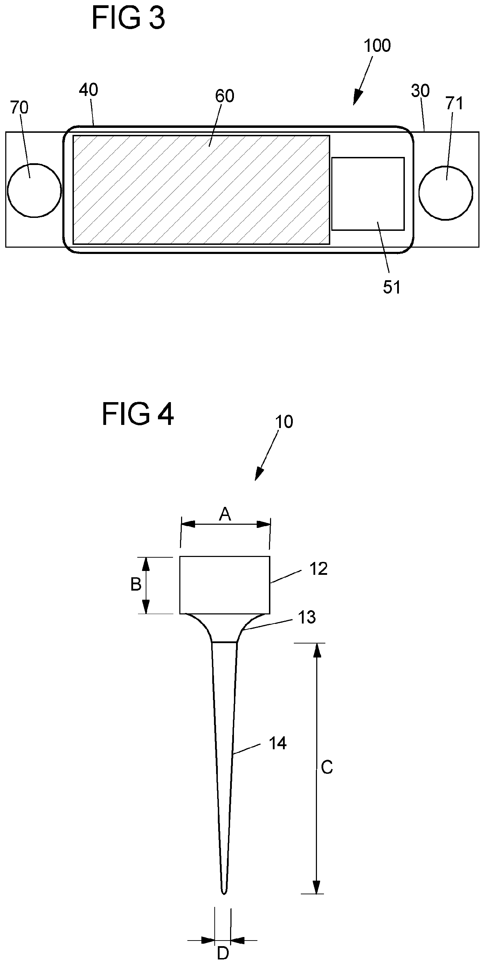

**Figure 3** illustrates one further embodiment of the device 100 in top view. Again, the chip 20, the sensor 51 and the coil are positioned on the interposer layer 30 and covered by the lid 40. The sensor 51 is a light source-based sensor. Further, on top of the lid 40 the far-file electrode 60 is positioned. The proximal end of each microneedle 10 is soldered by soldering points 11 to the surface of the chip 20, which ensures that each microneedle 10 of the array of a multiple of microneedles has a direct contact to the chip 20. Further the interposer layer 30 comprises two fixing holes 70, 71. Those fixing holes 70, 71 are positioned outside the lid 40 on each side of the lid 40 in the interposer layer 30. The fixing holes 70, 71 are suited to serve for the fixation of the device 100 into tissue. The device 100 can be fixated by screws, clamps or such like devices through the fixing holes 70, 71 in the interposer layer 30.

**Figure 4** illustrates one embodiment of a microneedle 10 of the array of microneedles. The microneedle 10 comprises a proximal end 12, a tapered portion 13 and a distal end 14, wherein the tapered portion 13 connects the proximal end 12 with the distal end 14. Accordingly the tapered portion 13 is as short as possible and serves only as connection between the proximal end 12 and the distal end 14. In one embodiment of the invention, the microneedle 10 according to the invention has a proximal end 12, which is shaped cylindrical with a diameter A between 0.05 mm and 0.5 mm, preferably the proximal end has a diameter A of 0.2 mm and a height B between 0.05 mm and 0.5 mm, preferably with a height B of 0.2 mm. The distal end 14 is needle shaped and has a length C between 0.5 mm and 2.0 mm. The distal end 14 of the microneedle 10 is electrically conductive and shear stress resistant in the range of 5 to 50 Newton, which is comparable to the shear stress resistance of bonding wires. Preferably the microneedle is milled from one piece. Preferably, the diameter D of the distal end 14 of the microneedle is between 0.001 mm and 0.1 mm, preferably between 0.01 mm and 0.1 mm, most preferably the diameters D of the distal end 14 of the microneedle is 0.02 mm. Thereby, the distal end 14 of the microneedle 10 approximates cellular dimensions.

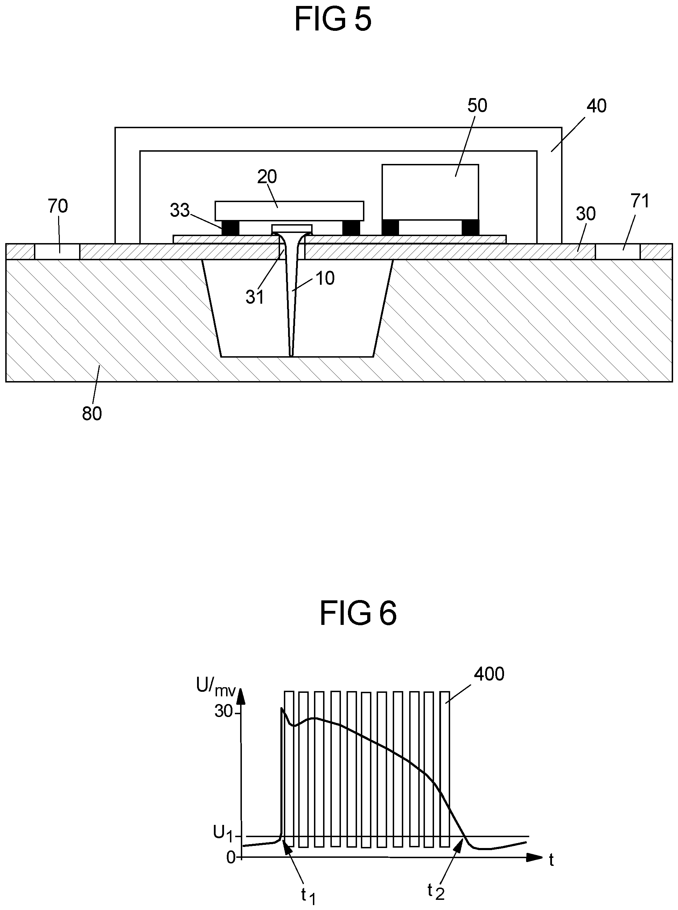

**Figure 5** illustrates one embodiment of the device 100. An interposer layer 30 is provided which is adapted to connect the microneedles 10 of the array of microneedles with the chip 20. Further, the interposer layer 30 comprises holes 31 which are suited for receiving a microneedle 10. Moreover, the interposer layer 30 comprises two fixation holes 70, 71. The interposer layer is covered by a temporary protective cover 80, which forms a sacrificial layer which is removed after assembly. A microneedle 10 is put inside the hole 31. The chip 20 is soldered to the interposer layer 30 by soldering points 33. A sensor 50 is additionally positioned on the interposer layer 30. Furthermore a coil is positioned on the interposer layer 30, which is not shown in the figure for the sake of clarity. The lid 40 is mounted to seal the device.

**Figure 6** illustrates the timing of the harvesting during an electrical cell event. The graph illustrates the amplitude of the cellular electrical activity over time during one electrical cell event as sensed over a singular microneedle 10 of the array of microneedles. As the amplitude of the cellular electrical activity reaches the reference level U₁ of the corresponding microneedle the cellular cycle time t₁ starts and harvesting cycles 400 are started. Energy harvesting is done until the amplitude of the cellular electrical activity falls below the reference level U₁ again, which is at time point t₂.

### Example 1

A device 100 according to the invention was build comprising an array of 7 x 17 microneedles 10 which are soldered to a chip 20 with a size of 3 x 7 mm and a height of 0.3 mm. The chip 20 with the array of microneedles was soldered to an interposer layer 30 with a size 4 mm x 15 mm. Next to the chip 20 a sensor 50 was positioned with a dimension of 2 x 2 mm. The chip 20, the sensor 50 were covered by a lid 40, wherein the lid had a size of 4 mm x 12 mm and the thickness of the lid-material was between 0.1 mm and 0.5 mm. Further, the interposer layer 30 comprises on each side of the lid 40 a fixation hole 70, 71. Each fixation hole 70, 71 had a diameter of 1.5 mm. The sensor 50 was a MEMS pressure sensor which was in touch with the lid 40, thereby the pressure transducer membrane is in contact with the surrounding. On top of the lid 40 a far-field electrode 60 with the size 3 x 7 mm was positioned.

### Example 2

An array of microneedles 10 was used in a device according to the invention, wherein each microneedle 10 had the following shape. Each microneedle 10 comprises a proximal end 12, a tapered portion 13 and a distal end 14, wherein the tapered portion 13 connects the proximal end 12 with the distal end 14. Accordingly, the tapered portion 13 is as short as possible and serves only as connection between the proximal end 12 and the distal end 14. The proximal end 12 was shaped cylindrical with a diameter A of 0.2 mm and a height B of 0.2 mm. The distal end 14 was needle shaped and had a length C of 1.5 mm. The diameter D of the distal end 14 of the microneedle was 0.02 mm. Thereby, the distal end 14 of the microneedle 10 approximates cellular dimensions.

### Example 3

A device 100 according to the invention was build comprising an array of 7 x 17 microneedles 10 which are soldered to a chip 20 with a size of 3 x 7 mm and a height of 0.3 mm. The chip 20 with the array of microneedles was soldered to an interposer layer 30 with a size 4 mm x 15 mm. Next to the chip 20 a sensor 51, which was a light source based sensor, was positioned with a dimension of 2 x 2 mm and a height of 1 mm. The chip 20, the sensor 50 were covered by a lid 40, wherein the lid had a size of 4 mm x 12 mm and the thickness of the lid-material was between 0.1 mm and 0.5 mm. The lid comprised a translucent material so that light was able to reach the light source based sensor. Further, the interposer layer 30 comprises on each side of the lid 40 a fixation hole 70, 71. Each fixation hole 70, 71 had a diameter of 1.5 mm. On top of the lid 40 a far-field electrode 60 with the size 3 x 7 mm was positioned.

### Reference List

- 10: microneedle
- 11: soldering point
- 12: proximal end
- 13: tapered portion
- 14: distal end
- 20: chip
- 21: wire
- 30: interposer layer
- 31: hole
- 33: soldering point
- 40: lid
- 50, 51: sensor
- 60: far-field electrode
- 70, 71: fixing hole
- 80: protective cover
- 100: device
- 400: harvesting cycle

## Claims

1. A device (100) to record signals, which comprises
a multiple of microneedles (10) forming an array of microneedles;
a chip (20) comprising at least one comparator with adaptive level, a sequence control circuit, at least one capacitor stack built by n capacitors and 2n switches, at least one buffer capacitor outside the at least one capacitor stack, at least two additional switches outside the at least one capacitor stack and a CMOS-Logic, wherein *n* ∈ *N*, wherein the n capacitors are adapted to be sequentially charged by at least one microneedle of the array of microneedles, which functions as DC input source, one after the other and wherein the 2n switches of the capacitor stack couple the n capacitors selectively to at least one microneedle of the array of microneedles and wherein the buffer capacitor outside the at least one capacitor stack is dedicated to be charged from the n capacitors of the capacitor stack at once;
an interposer layer (30) comprising holes (31) for the multiple of microneedles;
a lid (40);
at least one coil;
at least one sensor (50, 51);
wherein the chip (20), is located on one surface of the interposer layer (30);
wherein the lid (40) and the interposer layer (30) form a capsule for the chip (20) and the at least one coil;
wherein the capsule carries an outer Far-Field electrode (60);
wherein each microneedle (10) has a distal end (14) which protrudes from the chip (20); and
wherein the device (100) is adapted to be electrically self-sufficient due to harvesting of electrical energy on one or more microneedles from an electrical energy source of the surrounding.

2. A device (100) according to claim 1, **characterized in that** the device (100) further comprises at least one further capacitor.

3. A device (100) according to any of the preceding claims, **characterized in that** the at least one sensor (50, 51) is selected from the group comprising electrical pin-sensors, electrical Far-Field sensors, micromechanical (MEMS) activity sensors, electrostatical accelerometers, piezoceramic accelerometers, micromechanical (MEMS) pressure sensors, micromechanical (MEMS) temperature sensors, light source-based sensors.

4. A device (100) according to any of the preceding claims, **characterized in that** the device (100) comprises between 5 and 10000 microneedles (10), preferably between 25 and 1000 microneedles (10), most preferably between 100 and 250 microneedles (10).

5. A device (100) according to any of the preceding claims, **characterized in that** the electrical energy source is selected from the group comprising bioelectric signals, radio signals, thermal sources or vibrations.

6. A device (100) according to any of the preceding claims, **characterized in that** the device (100) further comprises an external interrogation/programmer unit.

7. A device (100) according to any of the preceding claims, **characterized in that** every microneedle (10) is adapted to be operable independent of the other microneedles (10).

8. A device (100) according to any of the preceding claims, **characterized in that** the diameters of the distal ends (14) of the multiple of microneedles (10) are between 0.001 mm and 0.1 mm, preferably between 0.01 mm and 0.1 mm, most preferably the diameters of the distal ends (14) of the multiple of microneedles (10) are 0.02 mm.

9. A device (100) according to any of the preceding claims, **characterized in that** the microneedles (10) comprise a material of the group comprising Platin/Iridium (Ptlr), gold, and fine metals.

10. A device (100) according any of the preceding claims, **characterized in that** each microneedle (10) is adapted to be able to harvest cellular energy.

11. A device (100) according any of the preceding claims, **characterized in that** each microneedle (10) is adapted to be able to harvest cellular energy and to sense intrinsic cellular activity.

12. Method for collection signals utilizing a device (100) according to any of claims 1 to 11, **characterized in that**
• at least one microneedle (10) of the array of microneedles is set to harvest energy and optionally at least one microneedle (10) of the array of microneedles is set to sense the amplitude of cellular electrical activity;
• energy is harvested and optionally the amplitude of cellular electrical activity is sensed at least by one microneedle;
• at least one sensor (50, 51) continuously records signals, which are stored and/or transferred to an external interrogation/programmer unit;
wherein harvested energy is used to operate the device.

13. Method according to claim 12, **characterized in that** more than one sensor (50, 51) continuously records signals.

14. Method according to claim 12 or 13, **characterized in that** the microneedles (10) of the array of microneedles are inserted into plant tissue and that energy is harvested from tissue cells.

15. Use of the device (100) according to any of claims 1 to 11 to monitor biological signals from plants, environmental parameters such as temperature, pressure, geometric coordinates (location), concentration of chemical substances, electrical, magnetic or electromagnetic fields in strength and orientation.

## Patentansprüche

1. Vorrichtung (100) zum Aufzeichnen von Signalen, die umfasst:
mehrere Mikronadeln (10), die eine Anordnung von Mikronadeln bilden;
einen Chip (20), umfassend wenigstens einen Komparator mit adaptivem Niveau, eine Sequenzsteuerungsschaltung, wenigstens einen Kondensatorstapel, der durch n Kondensatoren und 2n Schalter aufgebaut ist, wenigstens einen Pufferkondensator außerhalb des wenigstens einen Kondensatorstapels, wenigstens zwei zusätzliche Schalter außerhalb des wenigstens einen Kondensatorstapels und eine CMOS-Logik, wobei n E N ist, wobei die n Kondensatoren dafür ausgelegt sind, nacheinander durch wenigstens eine Mikronadel der Anordnung von Mikronadeln, die als DC-Eingangsquelle fungiert, sequenziell geladen zu werden und wobei die 2n Schalter des Kondensatorstapels die n Kondensatoren selektiv an wenigstens eine Mikronadel der Anordnung von Mikronadeln koppeln und wobei der Pufferkondensator außerhalb des wenigstens einen Kondensatorstapels dafür vorgesehen ist, von den n Kondensatoren des Kondensatorstapels auf einmal geladen zu werden;
eine Interposer-Schicht (30), umfassend Öffnungen (31) für die mehreren Mikronadeln;
einen Deckel (40);
wenigstens eine Spule;
wenigstens einen Sensor (50, 51);
wobei sich der Chip (20) auf einer Oberfläche der Interposer-Schicht (30) befindet;
wobei der Deckel (40) und die Interposer-Schicht (30) eine Kapsel für den Chip (20) und die wenigstens eine Spule bilden;
wobei die Kapsel eine äußere Fernfeldelektrode (60) trägt;
wobei jede Mikronadel (10) ein distales Ende (14) aufweist, das aus dem Chip (20) herausragt; und
wobei die Vorrichtung (100) dafür ausgelegt ist, elektrisch autark zu sein, indem elektrische Energie an einer oder mehreren Mikronadeln von einer elektrischen Energiequelle der Umgebung geerntet wird,

2. Vorrichtung (100) gemäß Anspruch 1, **dadurch gekennzeichnet, dass** die Vorrichtung (100) ferner wenigstens einen weiteren Kondensator umfasst.

3. Vorrichtung (100) gemäß einem der vorstehenden Ansprüche, **dadurch gekennzeichnet, dass** der wenigstens eine Sensor (50, 51) aus der Gruppe ausgewählt wird, die elektrische Stiftsensoren, elektrische Fernfeldsensoren, mikromechanische (MEMS) Aktivitätssensoren, elektrostatische Beschleunigungsmesser, piezokeramische Beschleunigungsmesser, mikromechanische (MEMS) Drucksensoren, mikromechanische (MEMS) Temperatursensoren, lichtquellenbasierte Sensoren umfasst.

4. Vorrichtung (100) gemäß einem der vorstehenden Ansprüche, **dadurch gekennzeichnet, dass** die Vorrichtung (100) zwischen 5 und 10000 Mikronadeln (10), vorzugsweise zwischen 25 und 1000 Mikronadeln (10), am stärksten bevorzugt zwischen 100 und 250 Mikronadeln (10) umfasst.

5. Vorrichtung (100) gemäß einem der vorstehenden Ansprüche, **dadurch gekennzeichnet, dass** die elektrische Energiequelle aus der Gruppe ausgewählt wird, die bioelektrische Signale, Funksignale, thermische Quellen oder Vibrationen umfasst.

6. Vorrichtung (100) gemäß einem der vorstehenden Ansprüche, **dadurch gekennzeichnet, dass** die Vorrichtung (100) ferner eine externe Abfrage-/Programmiereinheit umfasst.

7. Vorrichtung (100) gemäß einem der vorstehenden Ansprüche, **dadurch gekennzeichnet, dass** jede Mikronadel (10) dafür ausgelegt ist, unabhängig von den anderen Mikronadeln (10) betreibbar zu sein.

8. Vorrichtung (100) gemäß einem der vorstehenden Ansprüche, **dadurch gekennzeichnet, dass** die Durchmesser der distalen Enden (14) der mehreren Mikronadeln (10) zwischen 0,001 mm und 0,1 mm groß sind, vorzugsweise zwischen 0,01 mm und 0,1 mm; am stärksten bevorzugt ist ein Durchmesser der distalen Enden (14) der mehreren Mikronadeln (10) von 0,02 mm.

9. Vorrichtung (100) gemäß einem der vorstehenden Ansprüche, **dadurch gekennzeichnet, dass** die Mikronadeln (10) aus einem Material der Gruppe bestehen, die Platin/Iridium (Ptlr), Gold und Feinmetalle umfasst.

10. Vorrichtung (100) gemäß einem der vorstehenden Ansprüche, **dadurch gekennzeichnet, dass** jede Mikronadel (10) dafür ausgelegt ist, in der Lage zu sein, zellulare Energie zu ernten.

11. Vorrichtung (100) gemäß einem der vorstehenden Ansprüche, **dadurch gekennzeichnet, dass** jede Mikronadel (10) dafür ausgelegt ist, in der Lage zu sein, zellulare Energie zu ernten und intrinsische zellulare Aktivität zu erkennen.

12. Vorrichtung zur Sammlung von Signalen durch Nutzung einer Vorrichtung (100) gemäß einem der Ansprüche 1 bis 11, **dadurch gekennzeichnet, dass**
- wenigstens eine Mikronadel (10) der Anordnung von Mikronadeln dafür eingerichtet ist, Energie zu ernten und optional wenigstens eine Mikronadel (10) der Anordnung von Mikronadeln dafür eingerichtet ist, die Amplitude von zellularer elektrischer Aktivität zu erkennen;
- Energie geerntet wird und optional die Amplitude zellularer elektrischer Aktivität von wenigstens einer Mikronadel erkannt wird;
- wenigstens ein Sensor (50, 51) kontinuierlich Signale aufzeichnet, die gespeichert und/oder an eine externe Abfrage-/Programmiereinheit übertragen werden;
wobei die geerntete Energie verwendet wird, um die Vorrichtung zu betreiben.

13. Verfahren gemäß Anspruch 12, **dadurch gekennzeichnet, dass** mehr als ein Sensor (50, 51) kontinuierlich Signale aufzeichnet.

14. Verfahren gemäß Anspruch 12 oder 13, **dadurch gekennzeichnet, dass** die Mikronadeln (10) der Anordnung von Mikronadeln in Pflanzengewebe eingefügt werden und dass Energie aus Gewebezellen geerntet wird.

15. Verwenden der Vorrichtung (100) gemäß einem der Ansprüche 1 bis 11, um biologische Signale von Pflanzen, Umgebungsparameter wie etwa Temperatur, Druck, geometrische Koordinaten (Standort), eine Konzentration chemischer Substanzen, elektrische, magnetische oder elektromagnetische Felder hinsichtlich Stärke und Ausrichtung zu überwachen.

## Revendications

1. Dispositif (100) pour enregistrer des signaux, qui comprend
de multiples microaiguilles (10) formant un réseau de microaiguilles ;
une puce (20) comprenant au moins un comparateur avec un niveau adaptatif, un circuit de commande de séquence, au moins une pile de condensateurs constituée de n condensateurs et de 2n commutateurs, au moins un condensateur tampon à l'extérieur de l'au moins une pile de condensateurs, au moins deux commutateurs supplémentaires à l'extérieur de l'au moins une pile de condensateurs et une logique CMOS, dans lequel n ∈ N, dans lequel les n condensateurs sont conçus pour être chargés séquentiellement par au moins une microaiguille du réseau de microaiguille, qui fait office de source d'entrée de courant continu, les uns après les autres et dans lequel les 2n commutateurs de la pile de condensateurs couplent sélectivement les n condensateurs à au moins une microaiguille du réseau de microaiguilles et dans lequel le condensateur tampon à l'extérieur de l'au moins une pile de condensateurs est destiné à être chargé à partir des n condensateurs de la pile de condensateurs en une seule fois ;
une couche d'interposition (30) comprenant des trous (31) pour les multiples microaiguilles ;
un couvercle (40) ;
au moins une bobine ;
au moins un capteur (50, 51) ;
dans lequel la puce (20) est située sur une surface de la couche d'interposition (30) ;
dans lequel le couvercle (40) et la couche d'interposition (30) forment une capsule pour la puce (20) et l'au moins une bobine ;
dans lequel la capsule porte une électrode de champ lointain externe (60) ;
dans lequel chaque microaiguille (10) a une extrémité distale (14) qui dépasse de la puce (20) ; et
dans lequel le dispositif (100) est conçu pour être électriquement autosuffisant grâce à la collecte d'énergie électrique sur une ou plusieurs microaiguilles à partir d'une source d'énergie électrique environnante.

2. Dispositif (100) selon la revendication 1, **caractérisé en ce que** le dispositif (100) comprend en outre au moins un condensateur supplémentaire.

3. Dispositif (100) selon l'une quelconque des revendications précédentes, **caractérisé en ce que** l'au moins un capteur (50, 51) est choisi dans le groupe comprenant des capteurs à broches électriques, des capteurs de champ lointain électriques, des capteurs d'activité micromécaniques (MEMS), des accéléromètres électrostatiques, des accéléromètres piézocéramiques, des capteurs de pression micromécaniques (MEMS), des capteurs de température micromécaniques (MEMS), des capteurs à base de source lumineuse.

4. Dispositif (100) selon l'une quelconque des revendications précédentes, **caractérisé en ce que** le dispositif (100) comprend entre 5 et 10 000 microaiguilles (10), de préférence entre 25 et 1 000 microaiguilles (10), mieux encore entre 100 et 250 microaiguilles (10).

5. Dispositif (100) selon l'une quelconque des revendications précédentes, **caractérisé en ce que** la source d'énergie électrique est sélectionnée dans le groupe comprenant les signaux bioélectriques, les signaux radio, les sources thermiques ou les vibrations.

6. Dispositif (100) selon l'une quelconque des revendications précédentes, **caractérisé en ce que** le dispositif (100) comprend en outre une unité d'interrogation/programmation externe.

7. Dispositif (100) selon l'une quelconque des revendications précédentes, **caractérisé en ce que** chaque microaiguille (10) est conçue pour être exploitable indépendamment des autres microaiguilles (10).

8. Dispositif (100) selon l'une quelconque des revendications précédentes, **caractérisé en ce que** les diamètres des extrémités distales (14) des multiples microaiguilles (10) sont entre 0,001 mm et 0,1 mm, de préférence entre 0,01 mm et 0,1 mm, mieux encore les diamètres des extrémités distales (14) des multiples microaiguilles (10) sont de 0,02 mm.

9. Dispositif (100) selon l'une quelconque des revendications précédentes, **caractérisé en ce que** les microaiguilles (10) comprennent un matériau du groupe comprenant le platine/l'iridium (PtIr), l'or et des métaux fins.

10. Dispositif (100) selon l'une quelconque des revendications précédentes, **caractérisé en ce que** chaque microaiguille (10) est conçue pour pouvoir récolter l'énergie cellulaire.

11. Dispositif (100) selon l'une quelconque des revendications précédentes, **caractérisé en ce que** chaque microaiguille (10) est conçue pour pouvoir récolter l'énergie cellulaire et détecter l'activité cellulaire intrinsèque.

12. Procédé pour la collecte de signaux utilisant un dispositif (100) selon l'une quelconque des revendications 1 à 11, **caractérisé en ce que**
• au moins une microaiguille (10) du réseau de microaiguilles est définie pour collecter l'énergie et, éventuellement, au moins une microaiguille (10) du réseau de microaiguilles est définie pour détecter l'amplitude de l'activité électrique cellulaire ;
• l'énergie est collectée et, éventuellement, l'amplitude de l'activité électrique cellulaire est détectée au moins par une microaiguille ;
• au moins un capteur (50, 51) enregistre en continu des signaux, qui sont stockés dans et/ou transférés vers une unité d'interrogation/programmation externe ;
dans lequel l'énergie récoltée est utilisée pour faire fonctionner le dispositif.

13. Procédé selon la revendication 12, **caractérisé en ce que** plus d'un capteur (50, 51) enregistre des signaux en continu.

14. Procédé selon la revendication 12 ou 13, **caractérisé en ce que** les microaiguilles (10) du réseau de microaiguilles sont insérées dans un tissu végétal et l'énergie est récoltée à partir des cellules du tissu.

15. Utilisation du dispositif (100) selon l'une des revendications 1 à 11 pour surveiller des signaux biologiques provenant de plantes, des paramètres environnementaux tels que la température, la pression, des coordonnées géométriques (emplacement), une concentration de substances chimiques, des champs électriques, magnétiques ou électromagnétiques en termes d'intensité et d'orientation.
